(19) Europäisches Patentamt
European Patent Office
Office européen des brevets

(11) Publication number: **0 038 511**
**B1**

(12) **EUROPEAN PATENT SPECIFICATION**

(45) Date of publication of patent specification: **16.01.85**

(51) Int. Cl.$^4$: **A 61 K 37/22,** A 61 K 35/12

(21) Application number: **81102848.9**

(22) Date of filing: **14.04.81**

# (54) Wound healing compositions.

(30) Priority: **17.04.80 CH 2972/80**

(43) Date of publication of application:
**28.10.81 Bulletin 81/43**

(45) Publication of the grant of the patent:
**16.01.85 Bulletin 85/03**

(84) Designated Contracting States:
**AT BE CH DE FR GB IT LI LU NL SE**

(56) References cited:
**DE-A-1 910 715**
**DE-A-1 959 933**
**FR-A-2 157 994**
**FR-A-2 257 268**
**FR-A-2 320 760**
**FR-M- 1 951**
**US-A-2 710 294**
**US-A-2 912 359**
**US-A-3 128 268**
**US-A-3 953 290**
**US-A-3 973 001**
**US-A-4 054 557**
**US-A-4 139 611**

**THE MERCK INDEX, Ninth Edition 1976, Rahway, N.J. US page 659, no. 4859, "Insulin"**

(73) Proprietor: **Schäfer, Rolf, Dr.**
**Grabenmattstrasse 37**
**CH-4133 Pratteln (CH)**

(72) Inventor: **Schäfer, Rolf, Dr.**
**Grabenmattstrasse 37**
**CH-4133 Pratteln (CH)**

(74) Representative: **Blum, Rudolf Emil Ernst et al**
**c/o E. Blum & Co Patentanwälte Vorderberg 11**
**CH-8044 Zürich (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to materials in mammalian tissues and fluids which accelerate wound healing.

The use of topically or systemically applied drugs for accelerated healing processes of physically, chemically or physiologically induced tissue lesions, such as burns, surgery or ulcers, is described in the medicinal literature. For example, there are reports on anabolic steroids, vitamin A, vitamin K, zinc compounds, silver compounds or serum factors, e.g. factor XIII in blood coagulation, or high molecular compounds such as collagen or cartilage which accelerate the healing process of tissue injuries. There are also reports on the successful treatment of tissue injuries with crude extracts from aloe or with low molecular weight extracts from mammalian organs. Such plant or animal extracts contain complex mixtures of poorly defined material with low levels of the active ingredients and high levels of inorganic salts.

The present invention provides a low molecular weight compound from crude extracts of mammalian organs, blood serum, or plasma which accelerates the healing process of mammalian and human tissue injuries. The compound can be isolated in relatively concentrated and pure form and has been substantially identified.

This invention relates to compositions containing a substance which is present in biological material and accelerate regeneration of internal and external tissue lesions.

The active material is a glycosphingolipid of the structure:

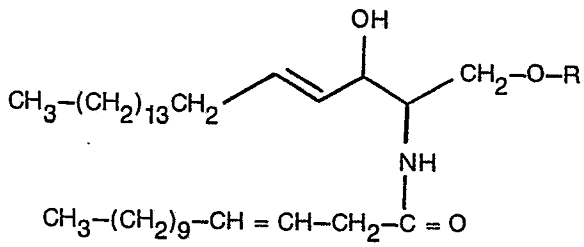

wherein R is an oligosaccharide having five sugar groups. This material can be isolated from mammalian tissues and fluids and promotes tissue formation in wounds after capillarization and vascularization begins.

The present invention provides compositions containing an active material which is isolated from mammalian tissues and fluids and which stimulates would healing. The compositions comprise a carrier and the active material of mammalian origin.

The active material is a glycosphingolipid of the structure:

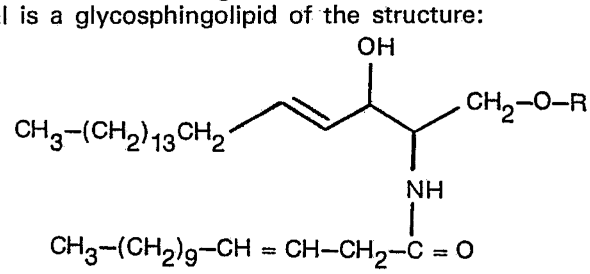

wherein R is an oligosaccharide having five sugar groups. This material can be isolated from mammalian tissues and fluids and promotes tissue formation in wounds after capillarization and vascularization begins.

It can be isolated from mammalian organs such as heart, lung, muscle and spleen or mammalian fluids, such as blood, plasma or serum. Relatively higher concentrations of the material is generally found in the organs. The mammals which have been found to produce the material are humans, swine, horses, sheep and bovine animals.

Isolation of the material can be accomplished by isolating an essentially protein-free fraction of the starting material having a molecular weight below 10,000 using a combination of techniques such as ultrafiltration, centrifugation and the like. Removal of the inorganic salts from the material may then be effected as by binding the active materials to active carbon. The material can then be further purified chromatographically. Details of exemplary means for isolating these materials are set forth below.

The maximum acceleration of wound healing is achieved when the glycosphingolipid is applied at a rate of 50 $\mu$g/ml of composition. Optimal rates of application are 5 $\mu$g/ml of the glycosphingolipid. Examples of suitable carriers include carboxymethyl cellulose, oil-in-water—or water-in-oil—emulsions, aqueous inorganic salt solutions or other conventional liquid, gel or ointment carrier materials.

The following example is illustrative of the invention.

Example

(a) Isolation of the glycosphingolipid from biological tissues

The glycosphingolipid of the invention is isolated in concentrated form from bovine heart according to the biochemical parting procedure described below.

25 kg of bovine heart were homogenized mechanically in the presence of 50 l of 0.2% NaCl solution at 4°C. Cellular debris was removed by centrifugation for 15 minutes at 10,000 xg. The clear supernatant was ultrafiltered at 4°C through dialysis tubes or ultrafilter membranes having an exclusion limit for molecules having a molecular weight greater than 10,000.

The ultrafiltrate was concentrated under vacuum at 40°C until the ionic strength of the solution corresponded to that of a physiological common salt solution (i.e. 0.8% NaCl). The organic material in the solution was bound to active carbon by suspending 200 g active carbon in the ultrafiltrate. The active carbon was centrifuged off after agitation with the solution for 30 minutes. Thereupon the active carbon was extracted with 100 ml 10% acetic acid or propionic acid. The solvent of the extract was removed under vacuum at 40°C and the residue was taken up in 100 ml 1M acetic acid.

The resulting acid solution was chromatographed over a molecular sieve, i.e., a 10-liter Biogel P 20® column (Biorad) and the column eluted with 0.2—2M acetic acid. After 1 column volume of eluate, the glycosphingolipid eluted. The solvent of the eluate of the lipid was removed under vacuum at 40°C.

The lipid portion was dissolved in a small volume of 0.1% acetic acid and charged on a silica gel absorption/desorption column covered with C-8 chains and equilibrated with 0.1% acetic acid. The column was then washed with a logarithmic gradient of 0.1% acetic to 90% ethanol. The lipid eluted with the 30% ethanol containing fraction.

The concentration of the lipid is shown in Table I.

TABLE I
Glycosphingolipid from bovine heart

| | Amount | Purification |
|---|---|---|
| Dry weight of the ultrafiltrate | 200 mg/ml | |
| Dry weight after C-8 silica gel column (calculated on the original volume of the ultrafiltrate) | 5 $\mu$g/ml | 40,000x |

The glycosphingolipid of the invention was purified exactly by the same procedure as described above when lung, muscle or spleen were the source materials. In case of blood, plasma or serum, the fluid was ultrafiltered directly and then processed as described above. Based on the amount of protein in the starting material, the recovery of the glyco-compound of the invention was 5—8 times higher in beef heart than in beef blood. The recovery of the glyco-compound from animal heart was of the same order of magnitude as from animal lung, muscle and spleen. The recoveries of the glyco-compound from human, swine, horses and sheep were of the same order of magnitude as from bovine animals.

(b) Biological activity of glycosphingolipid

In vitro the cell-proliferating activity of the glycosphingolipid was tested on primary human fibroblasts and epithelium cells damaged with THO (tritium water) for 24 hours. $^{14}$C-leucine incorporation in protein and $^{3}$H-thymidine incorporation in DNA was measured in the presence of whole medium (Eagles Medium) and 10 to 60 $\mu$g/ml medium of the lipid using a five-hour pulse $3 \times 10^6$ cells were present in each Petri dish of 5 cm diameter. The concentration of leucine and thymidine was 20 $\mu$M. The radioactivity was 1 $\mu$Ci/ml medium. Controls were treated similarly except for the absence of the lipid in the culture. Cell-proliferation data is compiled in Table II.

TABLE II

| | Counts/minute |
|---|---|
| Control | 15,300 |
| Glycosphingolipid 10 $\mu$g/ml | 28,731 |
| 20 ,, | 42,310 |
| 50 ,, | 71,430 |

In addition to increased $^{3}$H-thymidine incorporation it was observed that the THO-damaged fibroblasts and epithelium cells began to morphologically resemble normal fibroblasts and epithelium cells after 4 days action with as little as 5 $\mu$g/ml of the glycosphingolipid.

*In vivo* the cell-proliferating activities of the lipid was evidenced by the accelerated healing of punch and burn wounds on guinea pigs. A continuous treatment of punch and burn wounds on guinea pigs with an ointment containing 5 $\mu$g/ml of the glycosphingolipid resulted in a reduction of the healing time by 20%. An ointment containing 50 $\mu$g/ml of glyco-sphingolipid resulted in a reduction of healing time by 25—30%. For optimal results the ointment consisted of 2% carboxymethyl cellulose (Whatman), 2% propylene-glycol, 0.3% calcium lactate, 0.2% propylparaben and $5\times10^{-4}$% each of a mixture of lysine, glutamine, 2-purine and 2-pyrimidine nucleosides (cytidine, guanosine, adenosine and uridine).

Similar results were obtained when the carriers were water-in-oil or oil-in-water emulsion instead of a gel or when the glyco compound was dissolved in 0.8% NaCl-solution and applied to the wounds by aerosol spraying. The carrier alone without the glyco-compound was not active in the wound healing experiments.

$^{14}$C-leucine and $^{3}$H-thymidine pulse chase tests on guinea pigs were carried out and indicated that the glycosphingolipid promoted proliferation of fibroblasts and epithelial cells in the middle and end phase of healing.

The tissue regenerating properties of the glycosphingolipid were evaluated in double blind clinical trials on 25 humans with internal chronic skin ulcers induced after artery- and vein-plugs, after postphlebitis or ulcus cruris. In the trails two different carboxymethylcellulose gels were applied to the ulcers. Gel I contained 5 $\mu$g/g gel of the glycosphingolipid and gel II contained no active ingredient (placebo). The ulcers of the patients were treated alternating one week with gel I and the following week with gel II. The gels were applied twice a day. The tissue regenerating properties of the lipid were evaluated by planimetrical measurement of the surfaces of the individual ulcers once a week and are summarized in Table III. The results for the glycosphingolipid were excellent in 13 cases (regression >50%), good in 6 cases (regression between 25% and 50%), weak in 2 cases (regression <25%) and zero in 4 cases.

TABLE III

| Ulcer | Duration of treatment (weeks) | Percentage global regression of initial surface | Percentage amelioration due to Gel I | Gel II |
|---|---|---|---|---|
| 1 | 10 | 90.9 | 53.7 | 37.2 |
| 2 | 10 | 98.4 | 83.3 | 15.1 |
| 3 | 9 | 100 (cicatrisation complete) | 53.3 | 46.7 |
| 4 | 10 | 87.6 | 54.1 | 23.5 |
| 5 | 6 | 27.9 | 22.6 | 5.3 |
| 6 | 6 | 31.5 | 26.6 | 4.9 |
| 7 | 2 | aggravation | | |
| 8 | 2 | aggravation | | |
| 9 | 6 | 53.4 | 54.9 | −1.6 |
| 10 | 8 | 77.2 | 33.7 | 43.5 |
| 11 | 8 | 53.4 | 40.2 | 13.2 |
| 12 | 4 | aggravation | | |
| 13 | 4 | aggravation | | |
| 14 | 6 | 45.3 | 11.4 | 33.9 |
| 15 | 6 | 17.5 | 22.9 | −5.4 |
| 16 | 2 | 100 | 80 | 20 |
| 17 | 6 | 10.7 | 19.9 | 9.2 |
| 18 | 6 | 100 | 28.5 | 71.5 |
| 19 | 6 | 36.9 | 45.7 | −8.8 |
| 20 | 6 | 85.4 | 62.2 | 23.2 |
| 21 | 6 | 45.5 | 31.8 | 13.7 |
| 22 | 6 | 44.8 | 30.9 | 13.9 |
| 23 | 6 | 79.2 | 60.4 | 18.8 |
| 24 | 6 | 63.3 | 39.9 | 23.4 |
| 25 | 6 | 55 | 26.8 | 28.2 |

(c) Properties of glycosphingolipid

The structure of the lipid was determined as follows:

Incubation of the lipid in the presence of glycosidase or mild alkaline hydrolysis with sodium methylate resulted in the complete inactivation of its biological activity. The residual skeleton of the lipid was determined to be a ceramide by TLC and specific staining methods. Anhydrous methanolysis in the presence of HCl (amide cleavage) followed by coupled GC-MS spectrometry indicated presence of a fatty acid residue having the structure

$$R_2{=}CH_3{-}(CH_2)_9{-}CH{=}CH{-}CH_2{-}$$

and sphingosin. Incubation of the lipid in the presence of $OsO_4$ produced an oligosaccharide and ceramide. The ceramide was then treated with periodate. Coupled GC-MS spectrometry resulted in the identification of a fatty acid residue with the following structure

$$R_1{=}CH_3{-}(CH_2)_{13}{-}CH_2{-}.$$

When the oligosaccharide, which was separated from ceramide, was treated by mild periodate oxidation, 5 sugar residues could be identified: uronic acid, fucose and 3 modified and substituted hexoses.

Based on the above analytical procedure, the structure of the lipid was concluded to be:

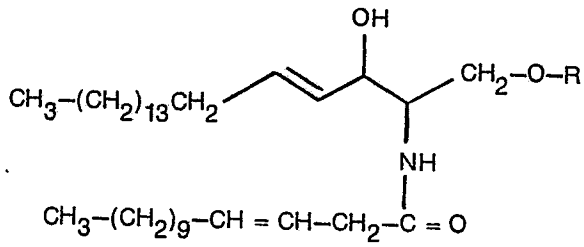

That is, the lipid was determined to be a glycosphingolipid.

Spectrometric data

The fatty acid residue ($R_1$) after periodate cleavage was methylated in the presence of a methanolic HCl solution and analyzed by coupled GC-field absorption mass spectrometry.

The following m/e peaks were obtained:

270 representing the molecule peak (M)

M-31 for $CH_2{=}\overset{+}{O}H$

M-74 for $H_2C{=}\underset{\displaystyle OH}{\underset{|}{C}}{-}OCH_3^+$

and the typical $(D_nH_{2n-1})^+$-series of 29, 43, 57, 71, 85, 99, 113, 127, 141, 155 and 169.

The fatty acid residue ($R_2$) after methanolic amide cleavage (fatty acid methylester) was incubated in the presence of periodate followed by subsequent hydrolysis with aqueous HCl. Two carboxylic acids were identified by gas chromatography using reference carboxylic acids in parallel runs:

$$HOOC{-}CH_2{-}COOH$$

and

$$CH_3{-}(CH_2)_9{-}COOH.$$

After GC-field adsorption mass spectrometry both acids gave identical fragmentation when compared with the corresponding reference carboxylic acid. When the $R_2$-methylester was analyzed by coupled GC-field adsorption mass spectrometry the following peaks were obtained:

240 for M

M-31 and M-74 for methoxy radicals

and again a typical $(C_2H_{2n-1})$ series.

**Claims**

1. A composition for stimulating wound healing comprising a glycosphingolipid and a carrier, said glycosphingolipid having the formula:

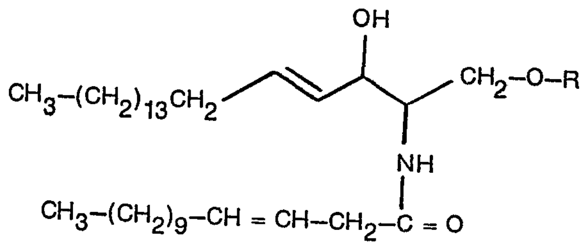

wherein R is an oligosaccharide containing five sugar units.

2. The composition of claim 1 wherein the carrier is selected from the group consisting of carboxymethylcellulose, oil-in-water emulsions, water-in-oil emulsions and aqueous solutions.

3. The composition of claim 1 wherein the glycosphingolipid is isolated from a mammalian tissue selected from the group consisting of heart, lung, muscle and spleen.

4. The composition of claim 1 wherein the glycosphingolipid is isolated from a mammalian fluid selected from the group consisting of blood, plasma and serum.

5. The composition of claim 1 wherein the glycosphingolipid is isolated from the fluid or organ of a mammal selected from the group consisting of humans, swine, horses, sheep and bovine animals.

6. The composition of claim 1 wherein the glycosphingolipid is isolated from mammalian tissue or fluid by:

(a) homogenizing the mammalian tissue or fluid with an aqueous solution with 0.2% NaCl;

(b) clearing the homogenate from cellular debris by centrifugation or filtration;

(c) thereafter ultra-filtering the cleared homogenate;

(d) concentrating the ultrafiltrate at 40°C until the ionic strength thereof correspond to a 0.8% NaCl solution;

(e) binding organic material in the concentrated ultrafiltrate to active carbon (1% by weight);

(f) thereafter extracting the active carbon with 10—30% acetic acid;

(g) evaporating the solvent from the extract;

(h) dissolving the residue in 0.2—2M acetic acid;

(i) chromatographing the resulting solution over a molecular sieve column;

(j) thereafter eluting the column with 0.2—2M acetic acid and collecting the first fraction with cell proliferating activity; and

(k) removing the solvent from the collected eluate;

(l) dissolving the dried eluate resulting from step (k) in acetic acid;

(m) adsorbing the dissolved eluate on an equilibrated silica gel column having C-8 chains;

(n) thereafter washing the column with a logarithmic gradient of 0.1% acetic acid—90% alcohol;

(o) collecting the 30% alcohol containing fraction; and

(p) evaporating the solvent.

## Patentansprüche

1. Eine Zusammensetzung für die Stimulierung der Wundheilung, dadurch gekennzeichnet, dass sie ein Glykosphingolipid und ein Trägermaterial enthält, wobei das genannte Glykosphingolipid die Formel

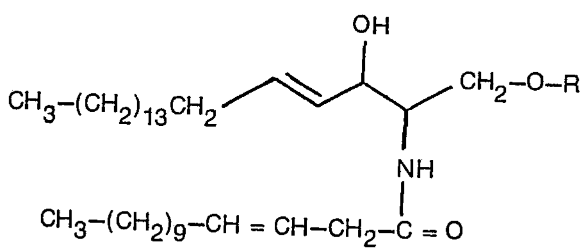

hat, worin R ein Oligosaccharid ist, das fünf Zuckereinheiten enthält.

2. Zusammensetzung nach Anspruch 1, dadurch gekennzeichnet, dass das Trägermaterial ausgewält ist aus der Gruppe, bestehend aus Carboxymethylzellulose, Oel-in-Wasser-Emulsionen, Wasser-in-Oel-Emulsionen und wässrigen Lösungen.

3. Zusammensetzung nach Anspruch 1, dadurch gekennzeichnet, dass das Glykosphingolipid aus einem Gewebe eines Säugers isoliert ist, ausgewählt aus der Gruppe, bestehend aus Herz, Lunge, Muskel und Milz.

4. Zusammensetzung nach Anspruch 1, dadurch gekennzeichnet, dass das Glykosphingolipid aus einer Flüssigkeit eines Säugers isoliert ist, ausgewählt aus der Gruppe, bestehend aus Blut, Plasma und Serum.

5. Zusammensetzung nach Anspruch 1, dadurch gekennzeichnet, dass das Glykosphingolipid aus

der Flüssigkeit oder einem Organ eines Säugers isoliert ist, ausgewählt aus der Gruppe, bestehend aus Menschen, Schweinen, Pferden, Schaften und Rindern und Ochsen.

6. Zusammensetzung nach Anspruch 1, dadurch gekennzeichnet, dass das Glykosphingolipid aus einem Gewebe oder einer Flüssigkeit eines Säugers wie folgt isoliert worden ist:

(a) Homogenisieren des Gewebes oder der Flüssigkeit des Säugers mit einer wässrigen Lösung mit 0,2% NaCl;

(b) Klären des Homogenates von zellulären Trümmern mittels Zentrifugation oder Filtration;

(c) anschliessendes Ultrafiltrieren des geklärten Homogenates;

(d) Konzentrieren des Ultrafiltrates bei einer Temperatur von 40°C bis die ionische Stärke davon einer 0,8% NaCl Lösung entspricht;

(e) Binden des organischen Materials im konzentrierten Ultrafiltrat an Aktivkohle (1 Gew.-%);

(f) anschliessendes Extrahieren der Aktivkohle mit 10—30% Essigsäure;

(g) Verdampfen des Lösungsmittels aus dem Extrakt;

(h) Auflösen des Restes in 0,2—2M Essigsäure;

(i) Chromatographieren der resultierenden Lösung über eine Molekularsiebsäule;

(j) anschliessendes Eluieren der Säule mit 0,2—2M Essigsäure und Sammeln der ersten Fraktion mit zell proliferierender Aktivität; und

(k) Entfernen des Lösungsmittels aus dem gesammelten Eluat;

(l) Auflösen des getrockneten Eluates, resultierend aus Schritt (k), in Essigsäure;

(m) Adsorbieren des aufgelösten Eluates an einer im Gleichgewicht stehenden Silikagelsäule, welche C-8 Ketten hat;

(n) anschliessendes Waschen der Säule mit einem logarithmischen Gradienten von 0,1% Essigsäure—90% Alkohol;

(o) Sammeln der 30% Alkohol enthaltenden Fraktion; und

(p) Verdampfen des Lösungsmittels.

**Revendications**

1. Composition pour stimuler la guérison et la cicatrisation de plaies comprenant un glycosphingolipide et un véhicule, le glycosphingolipide précité répondant à la formule suivante:

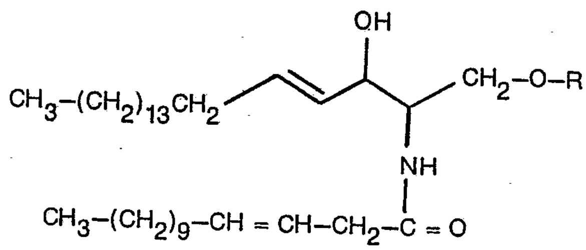

dans laquelle R représente un oligosaccharide contant cinq unités sucre.

2. Composition suivant la revendication 1, caractérisée en ce que le véhicule est choisi dans le groupe formé par la carboxyméthylcellulose, des émulsions huile-dans-eau, des émulsions eau-dans-huile et des solutions aqueuses.

3. Composition suivant la revendication 1, caractérisée en ce que le glycosphingolipide est isolé du tissu d'un mammifère choisi dans le groupe constitué du coeur, des poumons, des muscles et de la rate.

4. Composition suivant la revendication 1, caractérisée en ce que le glycosphingolipide est isolé à partir d'un liquide de mammifère choisi dans le groupe constitué du sang, du plasma et du sérum.

5. Composition suivant la revendication 1, caractérisée en ce que le glycosphingolipide est isolé d'un liquide ou d'un organe d'un mammifère choisi dans le groupe constitué des êtres humains, des porcs, des chevaux, des moutons et des bovidés.

6. Composition suivant la revendication 1, caractérisée en ce que le glycosphingolipide est isolé d'un liquide ou d'un tissue de mammifère par:

(a) homogénéisation du tissu ou du liquide du mammifère avec une solution aqueuse à 0,2% de NaCl;

(b) clarification de l'homogénéisat des débris cellulaires par centrifugation ou filtration;

(c) ensuite, ultrafiltration de l'homogénéisat clarifié;

(d) concentration de l'ultrafiltrat à 40°C jusqu'à ce que sa force ionique corresponde à une solution à 0,8% de NaCl;

(e) liaison de la matière organique présente dans l'ultrafiltrat concentré à du carbone actif (1% poids);

(f) ensuite extraction du carbone actif avec de l'acide acétique à 10—30%;

(g) évaporation du solvant de l'extrait;

(h) dissolution du résidu dans le l'acide acétique 0,2—2M;

(i) chromatographie de la solution ainsi obtenue sur une colonne à tamis moléculaires;

(j) ensuite, élution de la colonne avec de l'acide acétique 0,2—2M et collecte de la première fraction avec activité de prolifération des cellules;

(k) élimination du solvant de l'éluat recueilli;

(l) dissolution de l'éluat séché provenant de l'étape (k) dans de l'acide l'acétique;

(m) adsorption de l'éluat dissous sur une colonne de gel de silice équilibrée ayant des chaines en C-8;

(n) ensuite, lavage de la colonne avec un gradient logarithmique acide acétique à 0,1-alcool à 90%;

(o) collecte de la fraction contenant de l'alcool à 30%; et

(p) évaporation du solvant.